# EUROPEAN PATENT APPLICATION

(11) **EP 3 070 179 A1**
(43) Date of publication of application: **21.09.2016**
(21) Application number: 15382132.7
(22) Date of filing: 20.03.2015
(51) Int. Cl.: C12Q 1/68

(54) **GENETIC MARKERS AS INTRINSIC RISK FACTORS RELATED TO MUSCLE INJURIES**

(71) Applicant: Universitat de Barcelona, 08028 Barcelona (ES)
(72) Inventor: ARTELLS PRATS, Rosa, 08028 Barcelona (ES); PRUNA GRIVÉ, Ricard, 08328 Alella (ES)

(57) **Abstract**

The invention provides a process to predict the incidence of a subject for non-contact muscle injury, the recovery time of a subject from non-contact muscle injury, and/or the severity of a non-contact muscle injury of a subject, comprising determining, in a sample containing genetic material from the subject, the variant of a single nucleotide polymorphism (SNP) in an allele of the hepatocyte growth factor (HGF) gene of the subject.

SEQUENCE LISTING

<110>  Universitat de Barcelona

<120>  Genetic markers as intrinsic risk factors related to muscle
       injuries

<130>  AVCRI239

<160>  12

<170>  PatentIn version 3.5

<210>  1
<211>  51
<212>  DNA
<213>  Homo sapiens

<400>  1
atttttctac atgctggccc ttacccagct tttctgaatt tacaataaaa a                51


<210>  2
<211>  51
<212>  DNA
<213>  Homo sapiens

<400>  2
atttttctac atgctggccc ttacctagct tttctgaatt tacaataaaa a                51


<210>  3
<211>  51
<212>  DNA
<213>  Homo sapiens

<400>  3
cacttgcccc agtactgaga ccttacaaat ggcaatcttt ggaacactgt a                51


<210>  4
<211>  51
<212>  DNA
<213>  Homo sapiens

<400>  4
cacttgcccc agtactgaga ccttaaaaat ggcaatcttt ggaacactgt a                51


<210>  5
<211>  51
<212>  DNA
<213>  Homo sapiens

<400>  5
actttcatga accatgtagc ctcctaagaa tgcagtggct ggcttttggt a                51


<210>  6
<211>  51
<212>  DNA
<213>  Homo sapiens

<400>  6
actttcatga accatgtagc ctccttagaa tgcagtggct ggcttttggt a                51

<210>  7
<211>  51
<212>  DNA
<213>  Artificial Sequence

<220>
<223>  DNA probe

<400>  7
tttttattgt aaattcagaa aagctaggta agggccagca tgtagaaaaa t                51

<210>  8
<211>  51
<212>  DNA
<213>  Artificial Sequence

<220>
<223>  DNA probe

<400>  8
tttttattgt aaattcagaa aagctgggta agggccagca tgtagaaaaa t                51

<210>  9
<211>  51
<212>  DNA
<213>  Artificial Sequence

<220>
<223>  DNA probe

<400>  9
tacagtgttc caaagattgc catttgtaag gtctcagtac tggggcaagt g                51

<210>  10
<211>  51
<212>  DNA
<213>  Artificial Sequence

<220>
<223>  DNA probe

<400>  10
tacagtgttc caaagattgc catttttaag gtctcagtac tggggcaagt g                51

<210>  11
<211>  51
<212>  DNA
<213>  Artificial Sequence

<220>
<223>  DNA probe

<400>  11
actttcatga accatgtagc ctcctaagaa tgcagtggct ggcttttggt a                51

<210>  12
<211>  51
<212>  DNA
<213>  Artificial Sequence

<220>
<223>  DNA probe

<400>  12
actttcatga accatgtagc ctccttagaa tgcagtggct ggcttttggt a                51

## Description

The present invention relates to a method of predicting the incidence of a subject for non-contact muscle injury. The invention further relates to a method of predicting the time of recovery from non-contact muscle injuries of a subject. The invention also relates to a method of predicting the severity of non-contact muscle injuries of a subject. Further provided are uses of nucleic acid probes, primers and kits for these processes. The invention also provides a method of training an athlete.

### BACKGROUND ART

Sport has become increasingly popular both on a professional level as well as on the non-professional level. Injuries affect performance negatively and teams that can avoid injuries have greater success at the end of the season. Injuries are the main important adverse events during an athlete's career. Soccer is probably the most popular sport in the world. In soccer, muscle injuries represent between 20%-30% of all time loss injuries at professional level and up to 23% at amateur level. Approximately 16% of muscle injuries of professional soccer players are re-injuries. It has been shown. (cf. J. Ekstrand et al.; "Epidemiology of muscle injuries in professional football (soccer)"; The American Journal of Sports Medicine, 2011, vol. 39, p.p. 1226-1232) that an elite soccer team of 25 players can expect a mean of 15 muscle injuries per season with a mean absence time of 223 days, 148 missed training and 37 missed matches. In the same squad, only 0.4 anterior cruciate ligament ruptures per season can be expected, showing the great relevance of muscle injuries.

Muscles and muscle groups more frequently involved in muscle injuries of soccer players are the hamstrings (semitendinosus, semimembranosus and biceps femoris), quadriceps (rectus femoris and vastusmedialis, lateralis and intermedius) and the medial head of the gastrocnemius. Diagnosis is most commonly made clinically and imaging tools are used to identify the extent and site of the muscle injury. This information can be used as prognostic factor that could predict recovery time, return to pre-injury sport activity, and risk of recurrence.

Much is known about the multifactorial nature of muscle injuries in soccer as well as in other sports and this has led clinicians to develop exercise and rehabilitation programs and protocols according to sport-specific actions and movements. Programs are designed to treat and prevent muscle injuries, especially of players with prior injuries that are susceptible to recurrence. Injuries and re-injuries are important for elite athletes where decisions regarding return to sport and sport performance can have significant financial or strategic consequences for the individual sportsman and the team. The injury rate (IR) for ligament injuries decreased during the years 2000 but the IR for muscle injuries has remained high despite the prevention protocols.

### SUMMARY OF THE INVENTION

It is an object of the invention to provide a process or method of predicting the incidence of a subject for non-contact muscle injury, the recovery time of a subject from non-contact muscle injury, and/or the severity of a non-contact muscle injury of a subject. It is another object to provide a method of muscle injury analysis by which one can obtain information on muscle injury incidence, muscle injury severity, and an estimate of the recovery time from a muscle injury. This information would be useful in order to individualize programs for preventing injuries and to optimize the therapeutic and rehabilitation process after muscle injuries for minimizing time loss and for applying personalized preventive protocols to reduce the risk of re-injuries.

For solving the above objects, the invention provides a process of predicting the incidence of a subject for non-contact muscle injury, the process comprising determining a variant of a single nucleotide polymorphism (SNP) in an allele of the hepatocyte growth factor (HGF) gene of the subject.

The invention also provides a process of predicting the recovery time of a subject from non-contact muscle injury, the process comprising determining the variant of an SNP in an allele of the HGF gene of the subject.

The invention further provides a process of predicting the severity of a non-contact muscle injury of a subject, the process comprising determining the variant of an SNP in an allele of the HGF gene of the subject.

The invention further provides a process of predicting the incidence of a subject for non-contact muscle injury, the recovery time of a subject from non-contact muscle injury, and/or the severity of a non-contact muscle injury of a subject, comprising determining, in genetic material contained in a sample obtained from the subject, the variant of an SNP in an allele of the HGF gene of the subject; and associating the variant of the SNP of the HGF gene determined in step (ii) with a prospective decreased or increased incidence for non-contact muscle injury, with a prospective decreased or increased recovery time of the subject for non-contact muscle injury, and/or with a prospective severity of a non-contact muscle injuries of the subject.

The invention further provides a process of predicting the incidence of a subject for non-contact muscle injury, the recovery time of a subject from non-contact muscle injury, and/or the severity of a non-contact muscle injury of a subject, comprising:
(i) obtaining, from the subject, a sample containing genetic material of the subject;
(ii) determining, in the genetic material contained in the sample, the variant of an SNP in an allele of the HGF gene of the subject; and
(iii) associating the variant of the SNP of the HGF gene determined in step (ii) with a prospective decreased or increased incidence for non-contact muscle injury, with a prospective decreased or increased recovery time of the subject for non-contact muscle injury, and/or with a prospective severity of a non-contact muscle injuries of the subject.

The invention further provides a process of predicting the incidence of a subject for non-contact muscle injury, the recovery time of a subject from non-contact muscle injury, and/or the severity of a non-contact muscle injury of a subject, comprising associating the variant of an SNP of the HGF gene with a prospective decreased or increased incidence for non-contact muscle injury, with a prospective decreased or increased recovery time of the subject from non-contact muscle injury, and/or with an increased or decreased likelihood for a severe non-contact muscle injury of the subject, respectively.

The invention further provides a use of a kit of parts for predicting the incidence of a subject for a muscle injury, for predicting the time of recovery from non-contact muscle injuries of a subject, or for predicting the severity of non-contact muscle injuries of a subject, the kit comprising, as nucleic acid probes, optionally labeled nucleic acid molecules for determining, in genetic material obtained from the subject, a variant of an SNP in an allele of the HGF gene of the subject. The nucleic acid probe(s) may have a nucleotide sequence of any one or more of SEQ ID NOs 7 to 12 or may have a nucleotide sequence complementary to any one or more of those of SEQ ID NOs 7 to 12.

In one embodiment, the SNP in the HGF gene in the processes and kit mentioned above is selected from SNP1, SNP2 and SNP3 defined below.

The inventors have found that there is a correlation between injury incidence for non-contact muscle injuries, the severity and the recovery time from a non-contact muscle injury with SNPs in the HGF gene in a group of subjects. Using such statistical genetic information, one can predict the injury incidence, severity and recovery time for non-contact muscle injuries of an individual subject. Further, information on the SNPs in an individual can be used for a method of training and for optimizing the therapeutic and rehabilitation process of a subject after non-contact muscle injuries of the individual, whereby time loss for recovery can be minimized and personalized preventive protocols can be applied for reducing the risk of re-injuries.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention.

### BRIEF DESCRIPTION OF THE DRAWINGS

FIG 1. Severity of muscle injury according to genotypes of SNP1 (rs5745678).Y-axis: number of injuries; X axis: genotypes.Left bar (dark grey): mild; middle bar (light grey): moderate; right bar (grey): severe; p=0.002.
FIG 2. Estimated recovery time depending on variants of SNP2 (rs5745678); Y-axis: Recovery Time (Days, Mean, 95% IC); X-axis: genotypes; p=0.009.
FIG 3. Severity of muscle injury according to genotypes of SNP2 (rs5745697). Y-axis: number of injuries; X-axis: genotypes; left bar (dark grey): mild; middle bar (light grey): moderate; right bar (grey): severe; p=0.008.
FIG 4. Estimated recovery time depending on variants of SNP2 (rs5745697). Y-axis: Recovery Time (Days, Mean, 95% IC); X-axis: genotypes; p=0.02.
FIG 5. Severity of muscle injury according to genotypes of SNP3 (rs1011694). Y-axis: number of injuries; X-axis: genotypes; Left bar (dark grey): mild; middle bar (light grey): moderate; right bar (grey): severe; p=0.019.
FIG 6. Estimated recovery Time depending on variants of SNP3 (rs1011694). Y-axis: Recovery Time (Days, Mean, 95% IC); X-axis: genotypes; p=0.08.

### DETAILED DESCRIPTION OF PARTICULAR EMBODIMENTS

Muscle injuries are one of the most common traumas occurring in sport, most of which occurs by strain mechanism. The risk for muscle injuries is higher in high demand sports and muscle injuries account for a high percentage of all acute sports injuries. It is often difficult to predict long-term prognosis following a muscle strain, although these injuries may have a significant impact on the athletes and their teams. Injury diagnosis is usually clinical, and imaging tools can help to better understand extension and site of injury that could predict risk of recurrence, recovery time and return to sport activity. In recent years, in spite of intensive treatment programs and rehabilitation strategies, the number of muscle injuries remains high without reduction (cf. J. Ekstrand et al.; "Fewer ligament injuries but no preventive effect on muscle injuries and severe injuries: an 11-year follow-up of the UEFA Champions League injury study"; British Journal of Sports Medicine, 2013, vol. 47, p.p. 732-737). Muscle injuries may occur as a consequence of an interaction between extrinsic and intrinsic factors.

The non-contact muscle injuries considered in this invention are non-contact musculoskeletal tissue injuries (NCSMTIs), briefly referred to herein as "non-contact muscle injuries". Generally, the methods or processes of the invention are applied to subjects that have an increased likelihood of suffering from a non-contact muscle injury, e.g. due to sport activities. However, the invention is not limited to such subjects, as non-contact muscle injuries can also occur in everyday life of a subject. Herein, the subject generally is a mammal, preferably a human, more preferably the subject is a subject involved in competitive or high demand sports such as a human athlete. Among the types of competitive sports, there is no particular limitation and subjects performing team sports or individual sports can benefit from the invention. The correlations found in the present invention were obtained from a group of subjects in a team sport (soccer), since obtaining statistical data under controlled conditions on injury rates, recovery times or on the severity of injuries of a group of subjects is easier for a group of subjects involved in team sports. Among team sports of a subject of the invention, soccer (football), basketball, American football, and baseball are preferred, and soccer is most preferred. However, since the mechanisms by which muscle injuries can occur (strain) are not specific for a particular sport, the statistical data obtained from groups of subjects involved in team sports is not limited to application to subjects involved in the same or similar team sports, but can be used for making predictions for subjects in individual sporting disciplines, e.g. tennis players or sprinters, and other activities.

The incidence of a subject for non-contact muscle injury (herein briefly: "injury incidence" or "injury rate") is defined herein as the number of non-contact muscle injuries per exposure time, such as per 1000 hours exposure time. The type of exposure over the exposure time may be defined suitably depending on the group to which the subject belongs, such as depending on the type of activity of a subject e.g. the type sport. For subjects involved in sports, the exposure time preferably relates to the time the muscles of subjects are exposed to increased activity or stress (beyond activity and stress normally experienced everyday life), which may be the times of training and competition. Some sports associations use particular definitions of exposure time for particular sports; for example, the definition of UEFA (Union of European Football Associations) is found in: M. Hägglund et al.; "Methods for epidemiological study of injuries to professional football players: developing the UEFA model"; British Journal of Sports Medicine, 2005, vol. 39, p.p. 340-346. However, for practicing the present invention and for making the predictions of the invention, a definition of the type of exposure is not necessary, since the correlations disclosed herein may be used for the predictions of the invention for a subject without such definition.

The recovery time from a non-contact muscle injury is defined as the time between the occurrence of a non-contact muscle injury until the date for return to high activity of a subject such as full training and competition for an athlete.

The severity of a muscle injury may be classified as mild, moderate or severe based on magnetic resonance imaging (MRI) depending on the percentage of tissue damage. A mild muscle injury is defined in that less than 25% of the tissue of the muscle is affected, a severe injury is defined in that more than 50% of the tissue of the muscle is affected. Moderate muscle injuries are defined in that from 25% to 50% of the tissue of a muscle is affected. Alternatively, the severity of a muscle injury may be classified as mild (1-15 days), moderate (16-30 days) or severe (more than 30 days) according to the number of days that the subject such as a soccer player is absent from training or competition according to the Union of European Football Associations (UEFA) protocol.

A single nucleotide polymorphism (SNP) is a DNA sequence variation which occurs when a single nucleotide in the genome differs between paired chromosomes in a given individual or between individuals. SNPs can occur throughout the genome, both in coding and non-coding regions, and can affect the response of an individual to a specific treatment or other stimuli (cf. M. Monzo et al., "Genomic polymorphisms provide prognostic information in intermediate-risk acute myeloblastic leukemia"; Blood, 2006; vol. 107; p.p. 4871-4879).

In the present invention, SNPs in the HGF gene were identified, wherein the variants of the SNPs correlate statistically with the injury rate of a subject for non-contact muscle injuries, with the recovery time of a subject from non-contact muscle injuries and with the severity of non-contact muscle injuries. These correlations may be used in the invention for predicting, for individual subjects, the injury rate for non-contact muscle injuries, the recovery time from non-contact muscle injuries, and/or the severity of non-contact muscle injuries from the variant in one or more of the SNPs in an individual subject. Preferably, the injury rate for non-contact muscle injuries and/or the recovery time is predicted. More preferably, the injury rate is predicted.

The predictions that may be made according to the invention are generally relative predictions. The predictions are generally relative, since the properties to be predicted for a subject generally do not depend to the variant of the SNP alone, but generally depend on further factors such as extrinsic factors (e.g. type of training, weather conditions) and other intrinsic factors (e.g. age, sex, ethnic origin of subjects, earlier suffered injuries, diseases). Thus, while quantitative data on injury rate, recovery time or severity are generally used, and were used, for establishing a correlation as used in the invention, the predictions are generally not absolute with regard to injury rate, recovery time or severity, but relative. The predictions are generally relative compared to an average value of a control group of multiple subjects, wherein the control group comprises subjects having two or more genotypes of a given SNP. The control group should comprise subjects of multiple or all genotypes with regard to an SNP in the naturally occurring distribution among subjects. The control group may be a subgroup of subjects that is relevant for the individual subjects for which predictions are to be made. For example, the control group may comprise only subjects of Caucasian origin, of African or Hispanic origin. Generally, however, the control group will comprise subjects from various origins such as Caucasians and Africans or Caucasians, Africans and Hispanics. Other subgroups may be defined as control groups, such as females only or males only, as desired. The group of subjects used for establishing the correlations of the invention may be used as control group.

Accordingly, a prediction of increased or decreased injury rate predicts a higher or lower injury rate compared to a control group. Preferably, herein, the prediction on the injury rate based on an SNP of the invention has either one of the following two values: "decreased" or "increased".

Analogously, a prediction of increased or decreased recovery rate predicts a higher or lower recovery time compared to a control group. Thus, a prediction on the recovery time based on an SNP of the invention generally has either one of the following two values: "decreased" or "increased".

A prediction of the severity of a non-contact muscle injury may predict an increased or decreased likelihood for a severe non-contact muscle injury compared to a control group. Preferably, herein, the prediction on the injury severity based on an SNP of the invention has either one of the following two values: "decreased" or "increased". Medical personnel may use the predictions of the invention when planning therapies and rehabilitation of injured subjects.

The invention further provides a process of predicting the incidence of a subject for non-contact muscle injury, the recovery time of a subject from non-contact muscle injury, and/or the severity of a non-contact muscle injury of a subject, comprising associating the variant of an SNP of the HGF gene with a prospective decreased or increased incidence for non-contact muscle injury, with a prospective decreased or increased recovery time of the subject for non-contact muscle injury, and/or with an increased or decreased likelihood for a severe non-contact muscle injuries of the subject, respectively.

The inventors have surprisingly found SNPs in the HGF gene that are correlated with injury rate, recovery time and severity of muscle injuries. These SNPs may be from a non-translated region of the HGF gene. The variants of an SNP generally differ substantially with regard to their frequency, cf. Table 5. The variant (allele) of an SNP that is more frequent is referred to herein as "the more frequent variant", "wild-type allele" or "wild-type variant". The variant which is less frequent in a group or population is referred to herein as "the less frequent variant" or "minor variant". In one embodiment of the invention, the determined variant of an SNP of the HGF gene is that of a the less frequent variant of the SNP, and the presence of the less frequent variant of the SNP of the HGF gene is associated with an increased incidence of a subject for non-contact muscle injury and/or with a decreased prospective recovery time from non-contact muscle injury. In another embodiment, the determined variant of an SNP of the HGF gene is that of a the more frequent variant of the SNP, and the presence of the more frequent variant of the SNP of the HGF gene is associated with an increased likelihood of a severe non-contact muscle injury of the subject.

In another embodiment, the SNP of the HGF gene used in the invention is selected from SNP1, SNP2 and SNP3 defined in the following. SNP1 is rs5745678 in the NCBIdb database. SNP2 is rs5745697 defined in the NCBIdb database. SNP3 is rs1011694 in the NCBIdb database.These SNPs are defined as follows in the dbSNP database and the positions on chromosome 7 are indicated. The following sequences defining these SNPs correspond to the coding strand, i.e. the RNA-like strand, of the HGF gene (however, the complementary non-coding strand may also be used for determining the variant of an SNP):
rs5745678 (SNP1)
ATTTTTCTACATGCTGGCCCTTACC[C/T]AGCTTTTCTGAATTTACAATAAAAA Chromosome: 7:81742731
Functional Consequence: intron variant,utr variant 3 prime
Global MAF: A=0.1561/340

SEQ ID NO: 1 relates to the (more frequent) variant of SNP1 having the C nucleotide at the SNP position indicated by the square brackets. SEQ ID NO: 2 relates to the (less frequent) variant having the T nucleotide at the SNP position indicated by the square brackets.

rs5745697 (SNP2)
CACTTGCCCCAGTACTGAGACCTTA[A/C]AAATGGCAATCTTTGGAACACTGTA
Chromosome: 7:81728033
Functional Consequence: intron variant
Global MAF:T=0.1506/328

SEQ ID NO: 3 relates to the (more frequent) variant of SNP2 having the C nucleotide at the SNP position indicated by the square brackets. SEQ ID NO: 4 relates to the (less frequent) variant having the A nucleotide at the SNP position indicated by the square brackets.

rs1011694 (SNP3)
ACTTTCATGAACCATGTAGCCTCCT[A/T]AGAATGCAGTGGCTGGCTTTTGGTA
Chromosome: 7:81703677
Functional Consequence: intron variant
Global MAF: T=0.1267/276

SEQ ID NO: 5 relates to the (more frequent) variant of SNP3 having the A nucleotide at the SNP position indicated by the square brackets. SEQ ID NO: 6 relates to the (less frequent) variant having the T nucleotide at the SNP position indicated by the square brackets.

The coding strand of a HGF gene sequence having the wild-type bases at the positions of SNP1, SNP2 and SNP3 is shown in the ENSEMBL database for the HGF gene, entry ENSG00000019991,and can be accessed via the internet at the following address: http://www.ensembl.org/Homo_sapiens/Gene/Sequence?db=core;g=ENSG00000019991; r=7:81328322-81399754. SNP1 is at position 28308 and has the wild-type C at this position replaced by T in the less frequent variant. SNP2 is at position 43006 and has the wild-type C at this position replaced by A in the less frequent variant. SNP3 is at position 67362 and has the wild-type A replaced by T in the less frequent variant. These SNPs are known.

Herein, genotypes indicated by two bases designate the variants (alleles) in the diploid genome of a subject. Bases given for defining genotypes relate to the coding strand of the HGF gene. Accordingly, genotype "CC" with regard to SNP1 means the wild-type base C in both HGF alleles of the diploid genome, i.e. homozygosity for wild-type at the position of SNP1. Genotype "TT" with regard to SNP1 means the minor (less frequent) variant having a T at SNP1 in both HGF alleles of the diploid genome, i.e. homozygosity for the less frequent variant of SNP1. Genotype "CT" means heterozygosity for SNP1.

Genotype "CC" with regard to SNP2 means the wild-type base C in both HGF alleles of the diploid genome, i.e. homozygosity for the wild-type base at the position of SNP2. Genotype "AA" with regard to SNP2 means the less frequent variant of SNP2 having A in both HGF alleles of the diploid genome, i.e. homozygosity for SNP2. Genotype "CA" means heterozygosity for SNP2.

Genotype "AA" with regard to SNP3 means the wild-type base A of SNP3 in both HGF alleles of the diploid genome, i.e. homozygosity for the wild-type base at the position of SNP3. Genotype "TT" with regard to SNP3 means the minor (less frequent) variant of SNP3 having a T base in both HGF alleles of the diploid genome, i.e. homozygosity for SNP3. Genotype "AT" means heterozygosity for SNP3.

In the processes of predicting the incidence of a subject for non-contact muscle injury, the recovery time of a subject from non-contact muscle injury, and/or the severity of a non-contact muscle injury of a subject,the variant of an SNP in an allele of the HGF gene of the subject is determined or analyzed. Preferably, the variant of the SNP in both alleles in the diploid genome of a subject can be determined. With regard to the way the variant(s) of the SNP is/are determined, the invention is not limited and any of the methods known in the art may be used. If sequence information on alleles of the HGF gene of a subject, or other genetic information from which the variant of the SNP to be employed in the prediction may be derived, is already known, the determination of the variant may be done by analyzing the already known sequence or other genetic information.

In one embodiment, determining the variant of an SNP of the HGF gene comprises obtaining or taking, from the subject, a sample containing genetic material of the subject. There are no particular limitations regarding the source of the sample as long as it contains genetic material of the subject. For convenience of the subject, a blood sample and/or a saliva sample may be taken. The sample may be purified for reducing the content of components that are not genetic material. Preferably, DNA is isolated from the sample. Methods of isolating DNA from samples are known to the skilled person, and commercial kits may be employed for simplicity. The quality and quantity of the isolated DNA may be checked using a spectrophotometer. The DNA is then analyzed for the variant or variants of the SNP present in the sample, or the variants of multiple SNPs are determined. Preferably, the genetic material derived from the subject, such as the isolated DNA, is analyzed for the variant of SNP1, SNP2 and/or SNP3 in the HGF gene.

Many different methods are known that can be used for the determination step of the invention. Examples of SNP genotyping are hybridization-based methods (e.g. use of molecular beacons or use of SNP microarrays), enzyme methods (e.g. PCR-based methods or restriction fragment length polymorphism (RFLP) analysis or 5'-nuclease methods such as the TaqMan assay) and others. PCR-based methods are preferred. Preferably, real-time quantitative PCR is used. For example, a TaqMan assay may be used that may be combined with real-time quantitative PCR. As is known to the skilled person, the TaqMan assay may be performed concurrently with a PCR reaction and the results can be read in real-time as the PCR reaction proceeds. The assay uses forward and reverse PCR primers that amplify a region that includes the SNP site to be analyzed. The skilled person understands that the SNP may be determined on the coding or on the non-coding strand of the HGF gene, and probes used may be designed to hybridize to the coding or to the non-coding strand of the HGF gene.

In the TaqMan assay, allele discrimination can be achieved using FRET (fluorescence resonance energy transfer) signals combined with one or two allele-specific probes that hybridize to the SNP site. The probes generally used have a fluorophore linked to their 5' end and a quencher molecule linked to their 3' end. While the probe is intact, the quencher will remain in close proximity to the fluorophore, eliminating the fluorophore's fluorescence. During the PCR amplification step, if the allele-specific probe is perfectly complementary to the SNP allele, it will bind to the target DNA strand and then get degraded by 5'-nuclease activity of the Taq polymerase as it extends the DNA from the PCR primers. The degradation of the probe results in spatial separation of the fluorophore from the quencher molecule, generating a detectable signal due to thefluorophore's fluorescence. If the allele-specific probe is not perfectly complementary, it will have lower melting temperature and not bind as efficiently. This prevents the nuclease from acting on the probe. TaqMan probes have a fluorescence dye linked to their 5'-end and a quencher at the 3' end. The fluorescence of the fluorescence dyes on probes used in the same reaction tube should differ such that the emitted fluorescence originating from the dyes can be distinguished by the real-time PCR apparatus.

As described above, the presence or absence of a particular variant of an SNP may be followed by following the emitted fluorescence of the fluorescence dyes linked to and set free from the TaqMan probes. By using a probe for each variant of an SNP, homozygosity or heterozygosity for the SNPs can be determined and discriminated. In one embodiment, a probe for each of the variants of a particular SNP are used together in one reaction tube using different fluorescence dyes on the probes for detecting both variants in one and the same reaction tube. A sample from a subject may be analyzed for the presence or absence of SNP1, SNP2 and SNP3, which may be done in separate reaction tubes.

The genotype at one or more SNPs may then be associated with a prospective increased or decreased incidence for a non-contact muscle injury, with a prospective increased or decreased recovery time from a non-contact muscle injury, or with a prospective severity of a non-contact muscle injury of the subject.

The presence of the T allele of SNP1 may be associated with an increased incidence for non-contact muscle injury. In one embodiment, genotype CT or TT of SNP1 is associated with an increased incidence of muscle injury and/or the presence of genotype CC of SNP1 is associated with decreased incidence of non-contact muscle injury.

The presence of the A allele of SNP2 may be associated with an increased incidence for non-contact muscle injury. In more detail, genotype CA or AA of SNP2 is associated with increased incidence of non-contact muscle injury, and/or genotype CC of SNP2 may be associated with a decreased incidence of non-contact muscle injury.

The presence of the T allele of SNP3 may be associated with an increased incidence for non-contact muscle injury. In more detail, genotype AT or TT of SNP3 may be associated with an increased incidence of non-contact muscle injury, and/or genotype AA of SNP3 may be associated with a decreased incidence of non-contact muscle injury.

Further, the presence of the T allele of SNP1 may be associated with decreased recovery time from non-contact muscle injuries. In one embodiment, the CT or TT genotype is associated with decreased a recovery time from non-contact muscle injuries. Genotype CC of SNP1 may be associated with increased recovery time from non-contact muscle injuries.

The presence of the A allele of SNP2 may be associated with decreased recovery time from non-contact muscle injuries. In one embodiment, genotype CA or AA of SNP2 is associated with decreased recovery time from non-contact muscle injuries. The CC genotype at the position of SNP2 may be associated with increased recovery time from muscle injuries.

The presence of the T allele of SNP3 may be associated with decreased recovery time from non-contact muscle injuries. The AT or TT genotype of SNP3 may be associated with decreased recovery time from non-contact muscle injuries. The AA genotype of SNP3 may be associated with increased recovery time from non-contact muscle injuries.

Further, the presence of genotype CC of SNP1 may be associated with an increased likelihood for severe non-contact muscle injuries. Genotype CC of SNP2 may be associated with an increased likelihood for severe non-contact muscle injuries. Moreover, genotype AA of SNP3 may be associated with an increased likelihood for severe non-contact muscle injuries.

In the present invention, for increasing the reliability of the predictions according to the invention, two or all of the nucleotides at the positions of SNP1, SNP2 and SNP3 may be employed. In such case, variants of two or more SNPs, the predictions of which are the same increase the reliability of the prediction.

The predictions made according to the invention allow, inter alia, adaptations of the treatment and training if a non-contact muscle injury has occurred. For example, if a subject is determined to have a long recovery time, the training-free period may be extended compared to an average training-free period that would normally be used for a given type of a non-contact muscle injury of the control group. In contrast, the training-free period of a subject determined as having short recovery times after a muscle injury may be made shorter than the average period used for the control group in the absence of the knowledge obtained by using the present invention. Thus, the invention allows applying personalized preventive and/or recovery programs to a subject having a non-contact muscle injury that is more suitable for a particular subject than in the absence of the knowledge obtained according to the invention.

Accordingly, the invention also provides a method of training of an athlete, the method comprising determining the genotype at the positions of one or more SNPs of the HGF gene as described above; selecting exercises for the athlete depending on the genotype at one or more positions of SNP1, SNP2 and SNP3; and performing the selected exercises. Naturally, information other than that obtained from the process of the invention is generally also used for devising a training or recovery program for a subject, including the type of injury, other injuries that may be present, the type of sport practiced, and anthropometric information.

### Subjects and Injuries

Data was collected from 74 elite soccer players. These players suffered 220 muscle injuries during five consecutive seasons (2008-2009/2012-2013). Population characteristics were described in a previous study (cf. R. Pruna et al., "Single nucleotide polymorphisms associated with non-contact soft tissue injuries in elite professional soccer players Influence on degree of injury and recovery time"; BMC Musculoskeletal Disorders, 2013, vol. 14, p. 221).

Muscle injuries were diagnosed by clinical symptoms, physical examination and imaging using ultrasound techniques (US) and magnetic resonance imaging (MRI). Muscle injuries were classified as mild (1-15 days), moderate (16-30 days) or severe (more than 30 days) according to the number of days that the player is absent from training or competition according to the Union of European Football Associations (UEFA) protocol. US and MRI were used to classify injuries by anatomic region. Moreover, US and MRI gave information about the percentage of tissue damage; so, a mild injury represents a minimal damage under 25% and in a serious injury more than 50% of the tissue is affected. In case of moderate injuries the damaged tissue is close to 50%. Finally, the *recovery time* is defined as the date of the injury until the date of the players return to full training and competition. The *injury incidence* was defined as the number of injuries per exposure time (per 1000 hours). The study was approved an Ethical Committee of a hospital.

### DNA purification

Genomic DNA was obtained from 4 ml blood using NucleoSpin Blood (Macherey-Nagel, D, REF: 740951.250) following the standard protocol and was measured with a Nanodrop ND-1000 Spectrophotometer (TermoFisher Scientific, INC, Waltman, MA). Finally, it was stored at -80 °C until analyzed.

### SNP selection and primers and probes

Inventors have analyzed eight SNPs in genes related to connective tissue regeneration and repair (cf. the above-cited paper by R. Pruna et al.; and R. Pruna, et al., "The impact of single nucleotide polymorphisms on patterns of non-contact musculoskeletal soft tissue injuries in a football player population according to ethnicity"; Medicina clinica, 2015 Feb 2;144(3):105-10. doi: 10.1016/j.medcli.2013.09.026. Epub 2013 Dec 15). Probes were obtained from Applied Biosystems (AB, Foster City, CA) that are labeled with fluorescent probes FAM or VIC.

Each TaqMan® Predesigned SNP Genotyping Assay in the Life Technologies collection includes two allele-specific TaqMan® MGB probes containing distinct fluorescent dyes and a PCR primer pair to detect specific SNP targets. SNP Genotyping Assays contain a VIC® dye-labeled probe, a FAM™ dye-labeled probe, and two target-specific primers. TaqMan® probes incorporate MGB technology at the 3'-end to deliver superior allelic discrimination. The MGB molecule binds to the DNA helix minor groove, improving hybridization based assays by stabilizing the MGB probe-template complex. This increased binding stability permits the use of probes as short as 13 bases for improved mismatch discrimination and greater flexibility when designing assays for difficult or variable sequences. All MGB probes also include a non-fluorescent quencher (NFQ) that virtually eliminates the background fluorescence and provides excellent signal-to-noise ratio for superior assay sensitivity (TaqMan SNP genotyping Assay Handbook; https://tools.lifetechnologies.com/content/sfs/manuals/TaqMan_NP_enotypings_Assays _man.pdf).

### Genotyping

SNP analysis was done using a real-time PCR allelic discrimination TaqMan assay according to the manufacturer's (AB) instructions with minor modifications of the standard amounts of reagents. All PCR reactions were run in duplicate and contained 50 ng of DNA, 6.25 µL of Kappa Probe Fast Universal 2x qPCR Master Mix (REF: B4KK4702, Cultek), 0.25 µL of probes diluted 50% from the original concentration 40x (original volume and concentration: 188 µl, 40x; 0.25 µl at 20x concentration) is used, and water up to a final volume of 13 µL. Appropriate negative controls were also run. Real-time PCR was done on an ABI Prism 7500 Sequence Detection System (AB) using the following conditions: 50 °C for 2 min, 95 °C for 10 min, and then 40 cycles of amplification (95 °C for 15 s and 62 °C for 1 min). For each cycle, the software measures the fluorescent signal from the VIC- or FAM-labeled probe.

The following kits from Life Technologies may be used for SNP1, SNP2 and SNP3:
HGF_1: PN4351379; Lot Number: P140519-005 G08 (rs5745678)
HGF_2: PN4351379; Lot Number: P140424-007 G11 (rs5745697)
HGF_3: PN4351379; Lot Number: P130215-001 G03 (rs1011694)

The TaqMan probes employed for PCR in the TaqMan assay had the following nucleotide sequences (shown in 5' to 3' direction). The SNP positions are indicated by the base in square brackets.
For SNP1:
   TTTTTATTGTAAATTCAGAAAAGCT[A]GGTAAGGGCCAGCATGTAGAAAAAT (SEQ ID NO: 7), VIC-labeled
   TTTTTATTGTAAATTCAGAAAAGCT[G]GGTAAGGGCCAGCATGTAGAAAAAT (SEQ ID NO: 8), FAM-labeled
For SNP2:
   TACAGTGTTCCAAAGATTGCCATTT[G]TAAGGTCTCAGTACTGGGGCAAGTG (SEQ ID NO: 9), VIC-labeled
   TACAGTGTTCCAAAGATTGCCATTT[T]TAAGGTCTCAGTACTGGGGCAAGTG (SEQ ID NO: 10), FAM-labeled
For SNP3:
   ACTTTCATGAACCATGTAGCCTCCT[A]AGAATGCAGTGGCTGGCTTTTGGTA (SEQ ID NO: 11), VIC-labeled
   ACTTTCATGAACCATGTAGCCTCCT[T]AGAATGCAGTGGCTGGCTTTTGGTA (SEQ ID NO: 12), FAM-labeled

The skilled person understands that the determining step may, alternatively, be carried out with nucleic acid probes having a nucleic acid sequence complementary to any one or more of the nucleic acid molecules of SEQ ID NOs 7 to 12.

### Statistical analyses

Descriptive statistics of the main demographic variables of the studied population was calculated. Frequency tables were used for the distribution of the SNPs for the genes studied (cf. Table 5). The association between injury rate and SNP variants was determined by analysis of variance. The association between type and degree of injury and the SNP variants was determined by the Chi-square test and Fisher's exact test when necessary. Further, the association between injury recovery time and SNP variants was evaluated using multivariate analysis of variance. All statistical analysis was performed using SPSS version 14.0 for Windows (SPSS Inc., Chicago, IL). Significance was set at p≤0.05.

### Population

The main characteristics of the population were summarized in the two above-cited papers by R. Pruna et al. There are no significant differences between ethnics groups for these characteristics (median age, median weight, median height and median work load) (cf. Table 1, where work load is expressed as minutes in competition and training per player and season). All the players included in the study lived within 30 km of the training field and were thus subject to the same climate and environmental conditions. All the players had the same amount of work, the same diet and the same ergogenic aids. The training field, the playing fields and the injury prevention protocols were also identical for all the players. During four and a half season, a total of 220 muscle injuries were recorded and of these 140 were mild (63.6%), 76 were moderate (34.5%) and 4 were severe (1.9%).

### Injury incidence

Table 2 shows the association between all SNPs analyzed in the HGF gene with the injury rate (number of injuries/1000h of exposure). The variant at the position of SNP1 showed a correlation in that individuals with genotype CC showed a decreased number of injuries and individuals with genotype CT or TT showed an increased number of injuries (p=0.059). The variants at the position of SNP2 and SNP3 showed a correlation where individuals with wild-type genotypes (CC for HGF2 and AA for HGF3) showed a decreased number of injuries (p=0.042 and p=0.047, respectively).

### Severity of injury and recovery time

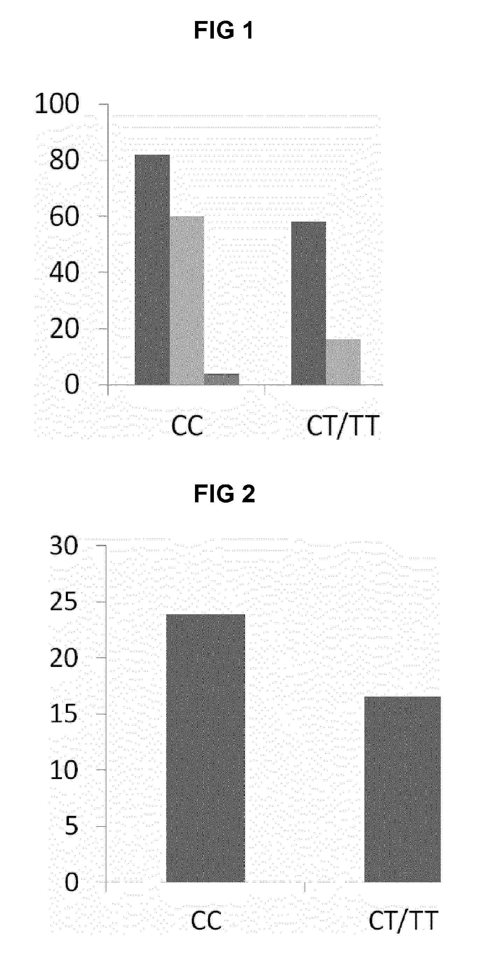

There is a correlation between the three SNPs analyzed in HGF (rs5745678 (HGF_1), rs5745697 (HGF_2) and rs1011694 (HGF_3)) with severity of injury and recovery time. At the position of SNP1, the presence of T allele, of both genotypes TT or TC, protects against the presence of severe injuries (p=0.002) and, moreover, this group represents a mean of recovery time of 19.83 days instead of 27.72 days in individuals with CC genotype (p=0.009) (cf. Table 3, Table 4, FIG 1 and FIG 2).

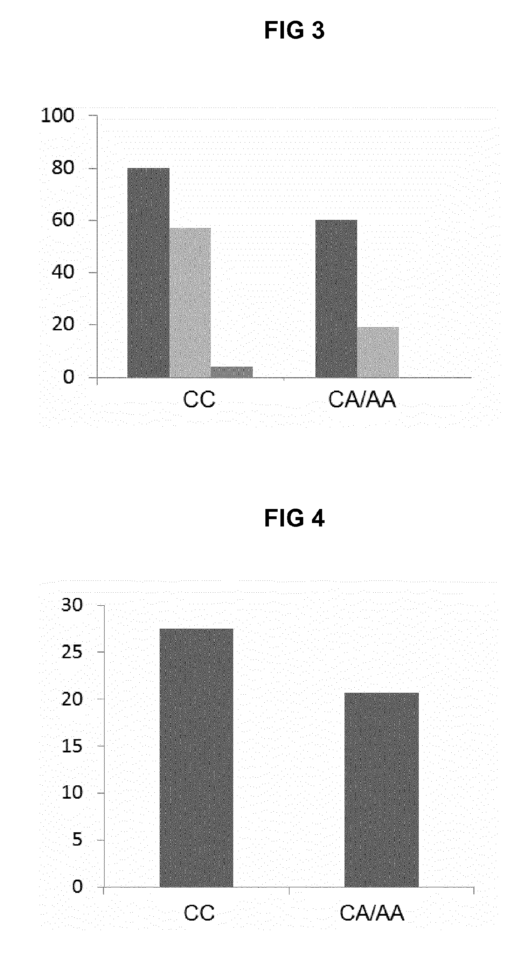

Injuries associated with the CA or AA genotype at the position of SNP2 (HGF_2) were not associated with severe injuries (p=0.008) and have a decreased recovery time (20.67 days) instead of 27.53 days in individuals with CC genotype (p=0.020) (cf. Table 3, Table 4, FIG 3 and FIG 4).

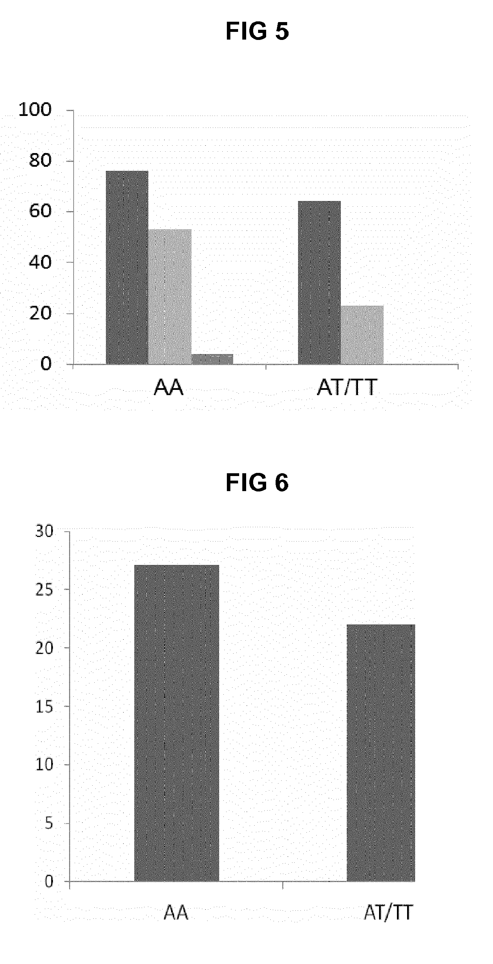

Injuries associated with genotypes AT or TT at the position of SNP3 (HGF_3) were mild or moderate but not severe (p=0.019) and a correlation was observed with recovery time (p=0.082), where injuries associated with AT or TT genotypes had a shorter mean recovery time (22.01 days) than those with the AA genotype (27.1 days) (cf. Table 3, Table 4, FIG 5 and FIG 6).

**Table 1: Main characteristics of the population studied**

| Characteristic | Whites | Black Africans | Hispanics |
|---|---|---|---|
| | n=42 (56.76%) | n=12 (16.22%) | n=20 (27.03%) |
| age | 25.52 (19-35) | 29.11 (20-35) | 26.16 (25-34) |
| weight | 76.44 (64-92) | 77.6 (64-92) | 72.63 (67-78.5) |
| height | 179.62 (166-195) | 181.3 (172-194) | 176.3 (169-189) |
| % fat | 8.66 (6.9-12.9) | 6.5 (6-14.1) | 10 (6.2-13.6) |
| work load | 15301 | 16224.3 | 16554.33 |

**Table 2: Genotypes related to injury rate**

| Gene | SNP | Genotype | Mean no. injuries/1000H (95%IC) | *p value* |
|---|---|---|---|---|
| HGF | rs5745678 | CC CT/TT | 8.5 injuries (95% IC:6.87-10.13) | p=0.059 |
| | | | 12.05 injuries (95% IC:7.53-16.58) | |
| HGF | rs5745697 | CC CA/AA | 8.4 injuries (95% IC:6.80-10.06) | p=0.042 |
| | | | 12.3 injuries (95% IC:7.78-16.73) | |
| HGF | rs1011694 | AA AT/TT | 8.4 injuries (95% IC:6.75-10.08) | p=0.047 |
| | | | 12.08 injuries (95% IC:7.89-16.28) | |

**Table 3: Genotypes related to muscle injury severity**

| Gene | SNP | Allel | Type of Muscle Injury Degree |
|---|---|---|---|
| HGF | HGF-1 | CC | Mild |
| | | | Moderate |
| | | | Severe |
| | | CT/TT | Mild |
| | | | Moderate |
| | | | - |
| | HGF-2 | CC | Mild |
| | | | Moderate |
| | | | Severe |
| | | CA/AA | Mild |
| | | | Moderate |
| | | | - |
| | HGF-3 | AA | Mild |
| | | | Moderate |
| | | | Severe |
| | | AT/TT | Mild |
| | | | Moderate |
| | | | - |

**Table 4: Genotypes related to recovery time**

| Gene | SNP | Genotype | Mean recovery time (days) (95% IC) | p value |
|---|---|---|---|---|
| HGF | rs5745678 | CC | 27.72 (23.88-31.57) | 0.009 |
| | | CT/TT | 19.83 (16.57-23.10) | |
| HGF | rs5745697 | CC | 27.53 (23.72-31.36) | 0.020 |
| | | CA/AA | 20.67 (16.96-24.38) | |
| HGF | rs1011694 | AA | 27.1 (23.11-31.03) | 0.082 |
| | | AT/TT | 22.01 (18.32-25.70) | |

**Table 5: Genotypic frequencies in the present study and for Caucasians (HapMap CEU), Black-African (HapMap YRI) and Hispanic (HISP1) populations in NCBI dbSNP**

| SNP (HGF gene) | Genotype | Population | | | | | |
|---|---|---|---|---|---|---|---|
| | | White | | Black African | | Hispanic | |
| | | Present Study | HapMap CEU | Present Study | HapMap YRI | Present Study | HapMap HISP |
| | | N=42 | | N=12 | | N=20 | |
| rs5745678 | CC | 30 (71.4%) | 58.3% | 11 (91.7%) | 100% | 10 (50%) | NA |
| | TT | - | 4.2% | - | - | 1 (5%) | |
| | CT | 12 (28.6%) | 37.5%% | 1 (8.3%) | - | 9 (40%) | |
| rs5745697 | AA | - | 2.7% | - | 0.9% | 1 (5%) | NA |
| | CC | 30 (71.4%) | 56.6% | 10 (83.3%) | 99.1% | 12 (60%) | |
| | CA | 12 (28.6%) | 40.7% | 2 (16.7%) | - | 7 (35%) | |
| rs1011694 | TT | | 1.7% | - | - | 1 (5%) | NA |
| | AA | 29 (69%) | 65% | 10 (83.3%) | 100% | 12 (60%) | |
| | AT | 13 (31%) | 33.3% | 2 (16.7%) | - | 7 (35%) | |

### List of nucleotide sequences of the sequence listing

SEQ ID NO: 1: frequent allele of SNP1
SEQ ID NO: 2: allele of minor frequent variant of SNP1
SEQ ID NO: 3: frequent allele of SNP2
SEQ ID NO: 4: allele of minor frequent variant of SNP2
SEQ ID NO: 5: frequent allele of SNP3
SEQ ID NO: 6: allele of minor frequent variant of SNP3
SEQ ID NO: 7: nucleotide sequence of TaqMan probe for SNP1, VIC-labeled
SEQ ID NO: 8: nucleotide sequence of TaqMan probe for SNP1, FAM-labeled
SEQ ID NO: 9: nucleotide sequence of TaqMan probe for SNP2, VIC-labeled
SEQ ID NO: 10: nucleotide sequence of TaqMan probe for SNP2, FAM-labeled
SEQ ID NO: 11: nucleotide sequence of TaqMan probe for SNP3, VIC-labeled
SEQ ID NO: 12: nucleotide sequence of TaqMan probe for SNP3, FAM-labeled

## Claims

1. A process of predicting the incidence of a subject for non-contact muscle injury, the recovery time of a subject from non-contact muscle injury, and/or the severity of a non-contact muscle injury of a subject, the process comprising determining, in a sample containing genetic material from the subject, the variant of a single nucleotide polymorphism (SNP) in an allele of the hepatocyte growth factor (HGF) gene of the subject.

2. The process according to claim 1, wherein the SNP is an SNP in a non-translated region of the HGF gene.

3. The process according to claim 1 or 2, wherein the SNP of the HGF gene is selected from the group consisting of SNP1, SNP2, and SNP3, these three SNPs being respectively defined by the following three NCBIdb database entries:
rs5745678, i.e. SEQ ID NO: 1 as more frequent variant and SEQ ID NO: 2 as less frequent variant;
rs5745697, i.e. SEQ ID NO: 3 as more frequent variant and SEQ ID NO: 4 as less frequent variant; and
rs1011694, i.e. SEQ ID NO: 5 as more frequent variant and SEQ ID NO: 6 as less frequent variant.

4. The process according to any one of claims 1 to 3, wherein the SNP is SNP1 and the presence of genotype CT or TT of SNP1 is associated with an increased incidence of non-contact muscle injury, and/or the presence of genotype CC of SNP1 is associated with a decreased incidence of non-contact muscle injury.

5. The process according to any one of claims 1 to 3, wherein the SNP is SNP2 and the presence of genotype CC of SNP2 is associated with a decreased incidence of non-contact muscle injury, and/or the presence of genotype CA or AA of SNP2 is associated with an increased incidence of non-contact muscle injury.

6. The process according to any one of claims 1 to 3, wherein the SNP is SNP3 and the presence of genotype AT or TT of SNP3 is associated with an increased incidence of non-contact muscle injury, and/or the presence of genotype AA of SNP3 is associated with a decreased incidence of non-contact muscle injury.

7. The process according to any one of claims 1 to 3, wherein the SNP is SNP1 and the presence of genotype TT or CT of SNP1 is associated with a decreased recovery time from non-contact muscle injuries, and/or genotype CC of SNP1 is associated with increased recovery time from non-contact muscle injuries.

8. The process according to any one of claims 1 to 3, wherein the SNP is SNP2 and the presence of the CA or AA genotype of SNP2 is associated with decreased recovery time from non-contact muscle injuries, and/or the presence of the CC genotype of SNP2 is associated with increased recovery time from non-contact muscle injuries.

9. The process according to any one of claims 1 to 3, wherein the SNP is SNP3 and the presence of the AT or TT genotype of SNP3 is associated with a decreased recovery time from non-contact muscle injuries, and the presence of the AA genotype of SNP3 is associated with an increased recovery time from non-contact muscle injuries.

10. The process according to any one of claims 1 to 3, wherein the SNP is SNP1 and the presence of genotype CC of SNP1 is associated with an increased likelihood for severe non-contact muscle injuries.

11. The process according to any one of claims 1 to 3, wherein the SNP is SNP2 and the presence of genotype CC of SNP2 is associated with an increased likelihood for severe non-contact muscle injuries.

12. The process according to any one of claims 1 to 3, wherein the SNP is SNP3 and the presence of genotype AA of SNP3 is associated with an increased likelihood for severe non-contact muscle injuries.

13. The process according to any one of claims 1 to 12, wherein the subject is a mammal, preferably a human, and more preferably a human athlete.

14. Use of a kit of parts for performing a process as defined in any one of the claims 1 to 13, the kit comprising, as probes, a pair of optionally labeled nucleic acid molecules, or nucleic acid molecules complementary thereto, selected from the following pairs (a) to (c):
(a)
5'-TTTTTATTGTAAATTCAGAAAAGCTAGGTAAGGGCCAGCATGTAGAAAAAT (SEQ ID NO: 7)
5'-TTTTTATTGTAAATTCAGAAAAGCTGGGTAAGGGCCAGCATGTAGAAAAAT (SEQ ID NO: 8)
(b)
5'-TACAGTGTTCCAAAGATTGCCATTTGTAAGGTCTCAGTACTGGGGCAAGTG (SEQ ID NO: 9)
5'-TACAGTGTTCCAAAGATTGCCATTTTTAAGGTCTCAGTACTGGGGCAAGTG (SEQ ID NO: 10)
(c)
5'-ACTTTCATGAACCATGTAGCCTCCTAAGAATGCAGTGGCTGGCTTTTGGTA (SEQ ID NO: 11)
5'-ACTTTCATGAACCATGTAGCCTCCTTAGAATGCAGTGGCTGGCTTTTGGTA (SEQ ID NO: 12).

15. A method of training of an athlete comprising the steps of: (i) determining the genotype of one or more SNPs selected from SNP1, SNP2 and SNP3, as defined in claim 3; (ii) selecting exercises for the athlete depending on the genotype of one or more of SNP1, SNP2 and SNP3; and (iii) performing the selected exercises.
